# EUROPEAN PATENT APPLICATION

(11) **EP 4 635 460 A1**
(43) Date of publication of application: **22.10.2025**
(21) Application number: 24171208.2
(22) Date of filing: 19.04.2024
(51) Int. Cl.: A61F 13/15, A61F 13/534, A61F 13/53

(54) **ABSORBENT ARTICLE WITH ABSORBENT CORE ENVELOPED IN A SHAPED WEB OF CONTINUOUS MICROFILAMENTS AND PROCESS FOR MANUFACTURING**

(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention relates to an absorbent article comprising an absorbent core which is enveloped in one or more webs made of continuous micro-filaments. The micro-filament webs exhibit a width that varies along the length of the absorbent core. The present invention also relates to a process for manufacturing such an absorbent article.

## Description

### Field of the invention

The present invention relates to an absorbent article comprising an absorbent core which is enveloped in one or more webs made of continuous micro-filaments. The micro-filament webs exhibit a width that varies along the length of the absorbent core. The present invention also relates to a process for manufacturing such an absorbent article.

### Background

There is abundant art relating to absorbent articles comprising an absorbent core as well as manufacturing methods thereof.

Often, absorbent articles exhibit a non-rectangular shape so as to adopt well to users' anatomy and stay in place during use. Absorbent cores may also exhibit a non-rectangular shape, but for certain applications, such as when employing off-line formed cores, it is often preferred to use rectangular ones. Also, it is often desired to use absorbent cores of small dimensions, be it to increase comfort during wear time or be it to position the absorbency close to the bodily exudate releasing organs. Thus, users are used to shaped absorbent cores as being visible within the absorbent article. Further, the fixation of rectangular absorbent cores may be reduced due to the smaller size relative to the total article size.

Henceforth, there is a need for adapting the absorbent member with relatively small absorbent core in an absorbent article so as to have a non-rectangular shape.

To this end, the present invention provides the manufacturing of an absorbent pad and an absorbent pad, as may be used as such or in combination with other elements for forming an absorbent article, whereby the absorbent core is enveloped with a non-woven material with continuous fibers. The envelop is significantly larger than the absorbent core and has a non-rectangular shape with a varying width along the length of the absorbent core. This not only provides a distinct visual impression of a larger and shaped product to the user, but also improves maintaining the proper posting of a relatively small absorbent core during manufacturing and use.
article design and a process for the manufacture of such an article, which envelops an absorbent material in a non-woven web, which is larger than the absorbent material and exhibits a non-rectangular shape.

### Summary

In a first aspect, the present invention is an absorbent pad for an absorbent article with an absorbent core, enveloped in a core envelope web.

The absorbent core exhibits a length, width and thickness extension. It has a user-oriented surface and an opposite surface, a first and a second longitudinally extending core side margin, and a front and a rear cross-directionally extending core end margin.

The absorbent core comprises superabsorbent polymer (SAP) particles intermixed with fibers, preferably continuous polymeric filaments.

The absorbent pad further comprises at least one core envelope web, covering at least the user oriented and the opposite surface of the absorbent core, projecting laterally outwardly of the first and a second longitudinally extending core side margin, thereby forming longitudinally extending core envelope web margins and exhibiting a core envelope web width. The absorbent core envelope web is consisting essentially of continuous micro-filaments, preferably of the coaxial meltblowing type.

Further, the core envelope web width is varying along the length extension of the absorbent core. The absorbent pad may further comprise one or more elements selected from the group consisting of
- the absorbent core is enveloped by a single web overfolded around the longitudinal core side margins and overlapping on the user oriented or the opposite surface;
- the absorbent core is enveloped by a user oriented and an opposite surface web connected to each other at least laterally outwardly of the longitudinal core side margins of the absorbent core;
- the absorbent core exhibits an essentially rectangular shape;
- the absorbent core exhibits an absorbent core length that is shorter than the one of the envelope web(s).

The absorbent pad may further comprise one or more elements selected from the group consisting of
- the envelope web(s) comprise synthetic thermoplastic polymers, preferably selected from the group consisting of PP, PE, PES, or PVA, optionally colored;
- the absorbent core comprises superabsorbent material, preferably particulate superabsorbent material;
- the absorbent core comprises superabsorbent polymer (SAP) particles selected from the group consisting of polyacrylate-, cellulose-, or starch-based materials;
- the absorbent core is essentially free of cellulosic material;
- the absorbent core comprises an intermix of essentially continuous thermoplastic filaments and SAP particles;
- the absorbent pad further comprises an additional material layer positioned between the absorbent core and the absorbent core envelope web, preferably positioned on the user-oriented surface of the absorbent core.

In another aspect, the present invention is a process for forming an absorbent pad comprising an absorbent core and at least one envelope web. The process is operated along a machine direction and exhibiting a cross-machine direction, and comprises the steps of
a - providing an absorbent pad forming unit comprising
   at least one continuous filament web forming unit;
   at least one absorbent core supply unit for providing an absorbent core;
   at least one web transfer unit;
   optionally an additional material supply unit for providing an additional material layer;
b - forming a first continuous filament web as a first precursor web of an envelope web and depositing it on a web transfer device;
c - transferring the first precursor web towards the absorbent core supply unit;
d - providing an absorbent core by the absorbent core supply unit;
e - depositing the absorbent core onto the precursor web on a web transfer device;
f - optionally providing an additional material layer and depositing it onto the absorbent core;
g - forming an envelope for the absorbent core to form the absorbent pad by
   g1a - applying a second continuous filament web as a second precursor web and positioning this onto the absorbent core,
      or
   g1b - overfolding the first continuous filament web as a first precursor web,
      and
   g2 - bonding the envelop webs to each other at least in portions laterally outward of the core; h - feeding the absorbent pad to further processing units for manufacturing an absorbent article, and characterized in that it further comprises a step of
   z - adjusting the width of the absorbent core envelope web such that it varies along the length of the absorbent core aligned with the machine direction of the absorbent pad forming unit.

The step z) of adjusting the width of the absorbent core envelope web may be executed
- by adjusting the width of the continuous filament web as a first and second precursor web during step b) and/or ga1)
   and/or
- by separating lateral portions of the continuous filament web as a first and second precursor web, preferably further including recycling of the separated lateral portions, more preferably recycling the separated lateral portions into the filament forming unit.

The step d) of providing an absorbent core may comprise the steps of
d1a - providing a preformed core precursor on a preformed core precursor supply unit;
d1b - adjusting the size of the core precursor to the predetermined size of the absorbent core.

The step d) of providing an absorbent core may comprises the steps of
d2a - providing a core forming unit comprising
   - an SAP supply unit;
   - a filament polymer supply unit;
   - a polymer extruder;
   - a continuous filament forming unit;
   - an SAP-Filament intermixing unit;
d2b - forming continuous polymeric filaments from a filament polymer by the continuous filament forming unit;
d2c - intermixing the continuous polymeric filaments with SAP particles from the SAP supply unit in the SAP-filament intermixing unit.

### Brief description of the Figures

Fig. 1 to 3 shows alternative approaches for an absorbent pad according to the present invention.
Fig. 4 to 6 depict equipment suitable for execution the process for manufacturing an absorbent pad according to the present invention.

The figures are schematic only, and not to scale. Same numerals refer to same or equivalent features or elements, single (`) or multiple (", '", ...) apostrophes indicate duplicate features, such a left and right or front and back, etc.,

### Detailed description

In one aspect the present invention is an absorbent pad and in a second aspect a method for manufacturing an absorbent pad, which may be integrated into a manufacturing process of absorbent articles.

Within the present context, "absorbent article" is used herein to refer to consumer products whose primary function is to absorb and retain human exudates like urine, menses, or faeces. "Absorbent article" is used herein to refer to an absorbent article generally worn by infants or adults about the lower torso, and may comprise diapers of the "pants type", produced with a full encircling waist belt, or of the "open type" with closure elements by which a user or caretaker connects the front and rear waist portions of the article, or of the "pre-closed type" with closure elements pre-fixed during manufacturing, but adapted to be opened and re-closed by a user or caretaker. An absorbent article comprises or may essentially consist of an absorbent pad, as may be integrated into other elements, such as chassis elements, to fit around the waist of a wearer. Absorbent pads may not be intended to fit around the waist but are primarily positioned in the crotch region of a wearer, extending somewhat towards the front and/or rear waist region. Such pads, often used in the feminine hygiene filed, may be held in place by underwear, optionally by attachments means, such as lateral wings to be folded around the crotch portion of the underwear.

The absorbent pads according to the present invention are typically disposable, whereby the term "disposable" is used herein to describe absorbent pads which generally are not intended to be laundered or otherwise restored or reused as an absorbent article, e.g., they are intended to be discarded after a single use and may also be configured to be recycled, composted or otherwise disposed of in an environmentally compatible manner. Often, though not necessarily, the pads are discarded together with the chassis elements.

The term "web" is used herein to describe a material which is primarily two- dimensional (i.e., in cartesian coordinates in an XY plane) and whose thickness (in a Z direction) is relatively small (i.e., 1/10 or less) in comparison to its length (in an X direction) and width (in a Y direction). Nonlimiting examples of webs include a layer or layers of fibrous materials, nonwovens, films and foils such as polymeric films or metallic foils. These materials may be used alone or may comprise two or more dub-layers laminated together.

Within the present context, the term "fiber" refers to elongated fibers exhibiting a length to diameter ratio of at least about 3:1, often more than 10:1, or even over 100:1. Synthetic or man-made natural material-based fibers are typically formed from solidifying continuous filaments of molten or dissolved polymers, as may be homogenous or monocomponent polymers, or polymers mixtures, or which may form distinct cross-sectional regions within a filament, whereby the latter may create crimped or crimpable fibers. The term "filament" is interchangeably used for essentially continuous solidified fibers, whilst "fibers" are used to describe dis-continuous structures with a well determinable fiber length. A typical process for forming a web from essentially continuous filaments is known as "spunbonding", see e.g., US5935512 (K-C), creating essentially continuous filaments of a diameter in the range of about 1 to 50 µm, often between 15 to 35 µm.
"Micro-fibers" is used to describe fibers as made by meltblowing web formation such as known from e.g., US8017534 (K-C), whereby low viscosity polymers are extruded through a nozzle and attenuated by a high velocity airstream blown at an angle relative to the formed filament. This scatters the melt, solidifies it and breaks it up into dis-continuous, relatively short fibers forming a fibrous web.

More recently, coaxial meltblowing (CoAx) technology has gained particular interest, providing essentially continuous micro filaments. Such CoAx filaments are formed by extruding a molten polymer through an array of nozzles, whereby the polymer filament exits from the tip of a capillary and is the shrouded by an annular air stream, as described in more detail in e.g., US9303334 (Biax). Whilst the polymers for such CoAx filaments can be made from a wide selection, preferred ones are polyester or polyolefins, especially polyolefins, and such as polypropylene. Whilst elastomeric polyolefins may be employed, this is not necessary from a performance point of view and less preferred from a commercial point of view due to their increased cost. If any thereof be employed, this should be at a level of less than 5 w-%, based on the total weight of the filament polymer. Preferred polymers exhibit a Melt Flow Rate MFR from 25 MFR of more than about 25 g/10 mins, preferably more than about 45 g/ 10 mins, typically less than about 2000 g/10 mins, as may be determined by ASTM D1238 and ISO 1133, and for polypropylene as a polymer that suitably can be processed with the current equipment and process, it is suitably expressed in units of gram per 10 minutes at 210°C and 2.16 kg load. When employing such filaments in an absorbent core, it may be preferred to select more resilient polymers, such as polyester.

Within the present context, a "fibrous or filamentary web" comprises a matrix of single type of fibers or filaments, or of a mixture, as may be directionally or randomly oriented, and bonded by friction, and/or adhesion, and/or cohesion, whereby the latter may be imparted directly after filament formation, e.g., at the lay-down step of fiber or filament forming. Typically, a fibrous web is self-supporting and allows handling on manufacturing or converting equipment, though just a sub-web of a composite web may not have sufficient integrity alone and ay be supported by a support equipment, such as a vacuum belt system and/or a support material, such as another web, as may be produced separately (off-line) and delivered as a roll good, or which may be formed in-line in an upstream (sub-)web-forming process step.

A web may comprise particles as a fiber- or filament-particle-intermixture. A "composite web" refers to a combination of (sub-)webs in a layered configuration, and a "continuous web" is essentially endless in the length or x-direction corresponding during manufacturing to machine direction (MD), as may be wound on rolls, or spools, or "festooned" in boxes, which may be spliced together to form the essentially endless web. Thus, a continuous web exhibits a width or y-direction perpendicular to the length or x-direction, corresponding to the cross-direction during manufacturing.

In the context of a discrete structure for an absorbent article or pad, the length direction or "longitudinal" means a direction running substantially from the front waist portion of a wearer through the crotch region to the rear waist region in a generally U-shaped in-use configuration. In the manufacturing configuration, the structure or the article be essentially flat or folded. Directions within 45 degrees of the longitudinal direction are considered to be "longitudinal." "Lateral" or widthwise refers to a direction running from a longitudinally extending side edge to a laterally opposing longitudinally extending side edge of an article - generally at a right angle to the longitudinal direction - and corresponds to a left-right orientation in the in-use configuration. Directions within 45 degrees of the lateral direction are considered to be "lateral." A term "discrete structure" applies to a discrete element, as may be cut off of a web, or may be formed as a discrete element e.g., in a forming drum.

The term "machine direction" (MD) is used herein to refer to the direction of material flow through a process. In addition, relative placement and movement of material can be described as flowing in the machine direction through a process from upstream in the process to downstream in the process along a manufacturing path. The term "cross direction" (CD) is used herein to refer to a direction that is generally perpendicular to the machine direction.

Within the present context, MD of a manufacturing equipment is generally aligned with the longitudinal direction of the web(s) or pad(s), and CD with the width direction thereof, although process variants may exist, where the absorbent core is turned by 90° before combined with an envelope web. However, a path of a web or structure may bend or turn, typically upward or downwardly along gravity, and the thickness-direction of the web or structure may change accordingly relative to gravity, but not relative to the width/length orientation of the structure. An absorbent pad according to the present invention as well as sub-structures or webs therein exhibit a basis weight, expressed in weigh per unit area (g/m²).

A "series of structures", such as absorbent pads, comprises a multiplicity of structures as follow - at least for a certain part of the manufacturing process - the same path on the manufacturing equipment.

The present disclosure relates to methods and apparatuses for manufacturing absorbent pads. During the manufacturing process, various continuous substrates and/or discrete components or structures may be combined with each other to form a continuous length of absorbent pads. At a downstream portion of the pad forming process, the continuous length of absorbent pads may be subjected to a separation step, such as a knife, to create separate and discrete absorbent pads.

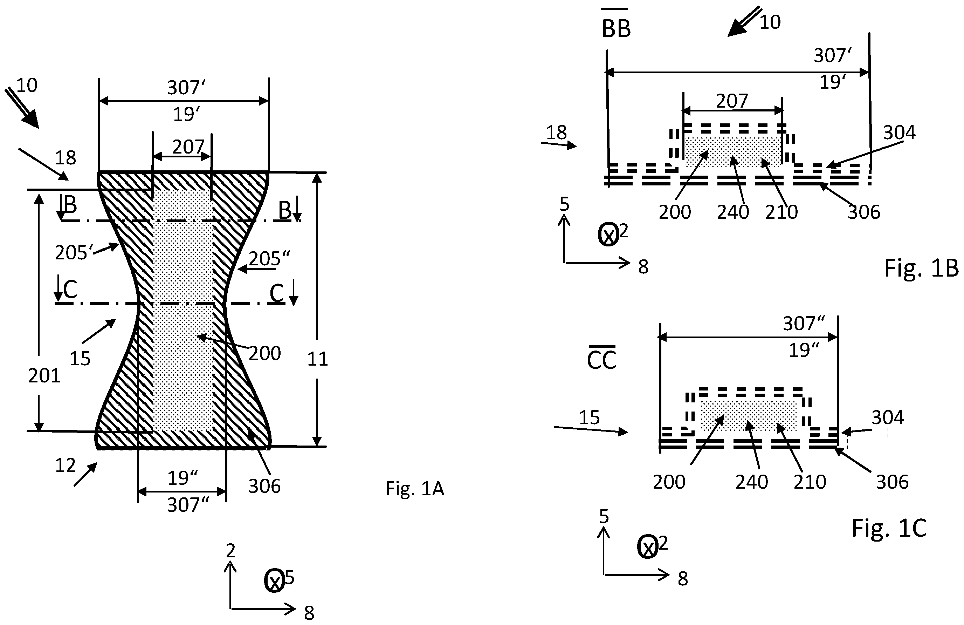

An absorbent pad includes a first surface, which is intended to be positioned towards a wearer during use, and a second surface opposite the first surface. A discrete absorbent article includes a first end and a second end, as well as a first end region, e.g., a front waist region, and a second end region, e.g., a rear waist region, separated from each other by a central region, e.g., a crotch region. For an absorbent pad according to the present invention it is important that an absorbent core comprising fibers and superabsorbent polymer (SAP) particles is enveloped in an envelope web of continuous micro-filaments, whereby the envelope web extends laterally outwardly of the absorbent core at an envelope web width which varies along the length of the absorbent core. The principles of such an absorbent pad 10 are now explained by referring to Fig. 1A to C, which should, however not be seen limiting. Fig. 1A depicts a top view of an absorbent core 200, here shown in a preferred rectangular shape, and positioned on an opposite envelope web 306, which extends in the lateral direction 8 outwardly of the absorbent core 200, and as non-limitingly seen also extending in the longitudinal direction 2 beyond the absorbent core 200. Thus, the shape of the absorbent pad 10 corresponds to the outer contour of the envelope web and a pad width to the width of the envelope web. Fig 1B depicts a cross-sectional view along line BB in Fig. 1A, being positioned in the rear portion 18 of the absorbent pad 10, showing the absorbent core 200 enveloped between the opposite envelope web 306 and the user oriented envelop web 304. The absorbent core 200 exhibits a core width 207 smaller than the width 307' of the envelope web, which corresponds to the article width 19' in the rear portion 18. Fig. 1C depicts a corresponding cross-sectional view, except that it is in the crotch region 15 of the absorbent pad 10 along line CC. There, the envelope web width 307" corresponding t the pad width 19" is different from the rear portion, here shown to be smaller.

Often it is desired that the absorbent core 200 exhibits a width that is relatively small so as to provide comfort to a wearer, but also to ease manufacturing. Thus, the width of an absorbent core 200 may be less than about 200 mm, or less than about 150 mm, or less than about 100 mm, but typically more than about 20 mm or more than about 50 mm, with a any of these dimensions referring to largest and smallest portion, respectively, although an essentially rectangular shape is often preferred.

An absorbent pad 10 according to the present invention may exhibit an overall pad length 11 that may be more than about 100 mm, such as for feminine hygiene articles or more than about 200 mm, or more than about 300 mm or more than about 400 mm or more than about 500 mm, as may be applicable for baby diapers, or more than about 600 mm or more than about 700 mm, or more than about 800 mm as may be appropriate for adult incontinence articles, but typically less than about 1500 mm. Further, the absorbent core exhibits a thickness, which may be as thin as more than about 1 mm, or more than about 3 mm, or more than about 5 mm or more than about 8 mm, or more than about 10 mm, but - due to comfort reasons, especially after the core is loaded - typically less than 20 mm, or less than 15 mm, all in the dry state.

The absorbent core 200 comprises superabsorbent polymers, preferably in particulate form, which are adapted to absorb liquids, such as water or aqueous solutions, such as bodily exudates, at multiples of their own weight. "Superabsorbent polymers" (SAP) refers herein to absorbent materials that can absorb at least 8 times their weight of an aqueous 0.9% saline solution as measured using the Centrifuge Retention Capacity (CRC) test (EDANA method NWSP 241.0.R2 (19)). The SAP preferably have a CRC value of at least 15 g/g. SAP are typically water-insoluble but water-swellable cross-linked polymers capable of absorbing large quantities of fluids. SAP are in particulate form so as to be flowable in the dry state. Typical particulate SAP are polyacrylate polymers, however it is not excluded that other polymer materials may also be used. For example, starch-based particulate absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile.

The particles may be relatively small (under 1 mm in their longest dimension) in their dry state and may be roughly circular in shape, but granules, fibers, flakes, spheres, powders, platelets and other shapes and forms are also known to persons skilled in the art.

The SAP may further be mixed with other dry additives, such as odour control material, antibacterial agents, such as without limitation active carbon, high surface area minerals, natural materials like herbs or herbal extracts, enzymatically active materials, or perfumes, all of which may be as such or be in a coated or micro-encapsulated form.

The absorbent cores according to the present invention comprises SAP particles 240 intermixed with fibers or filaments 230, preferably polymeric continuous micro-filaments, thus forming a fiber- or filament-particle intermixture. In a particular execution, the absorbent core is essentially free of cellulosic fibers.

As will be described in more detail herein below, the absorbent cores 200 may be pre-formed, such as in an off-line process and may be the provided in rolls, spools, or boxes, as well known in the art. Alternatively the absorbent cores 200 may be formed in-line, such as by extruding polymeric filaments and combining these with SAP particles.

In either case, the absorbent core 200 may require means to prevent SAP particles from migrating during manufacturing, transport or use. Henceforth, today such cores are often enveloped by various web materials, such a paper tissues or nonwoven webs, optimized for particle containment, fluid handling, i.e., not obstructing liquid flow, and cost. Typically, such envelope webs are supplied a single or a pair of roll goods and folded around the core or sandwiching it between two layers which are connected to each other such as by glue or other bonding techniques.

A particularly preferred execution comprises nonwoven webs of the above referenced continuous micro-filaments, preferably of the CoAx type. Further such envelop webs exhibit low basis weights of less than about 15 g/m², or less than about 10 g/m² or even less than about 5 g/m² though typically more than about 0.5 g/m².

Whilst smaller absorbent cores 200 are often preferred from a manufacturer perspective, consumers often connect "small core" with "low absorbency", even if the absorbent core comprises high liquid retention. Also, in particular if the absorbent core 200 has a small rectangular shape, this may yield to perceived or actual sub-optimal fit, as may result from incorrect placement upon donning or from dislocating during wear.

Henceforth it is an objective of the present invention to improve on the size and shape of the absorbent pad whilst maintaining a small size of the absorbent core. Thus, the present invention provides an absorbent pad 10 with an absorbent core 200 that is enveloped by an envelop web 300 of continuous micro-filaments that exhibit a shaped contour with a varying width 19', 19" along the length extension of the pad 10.

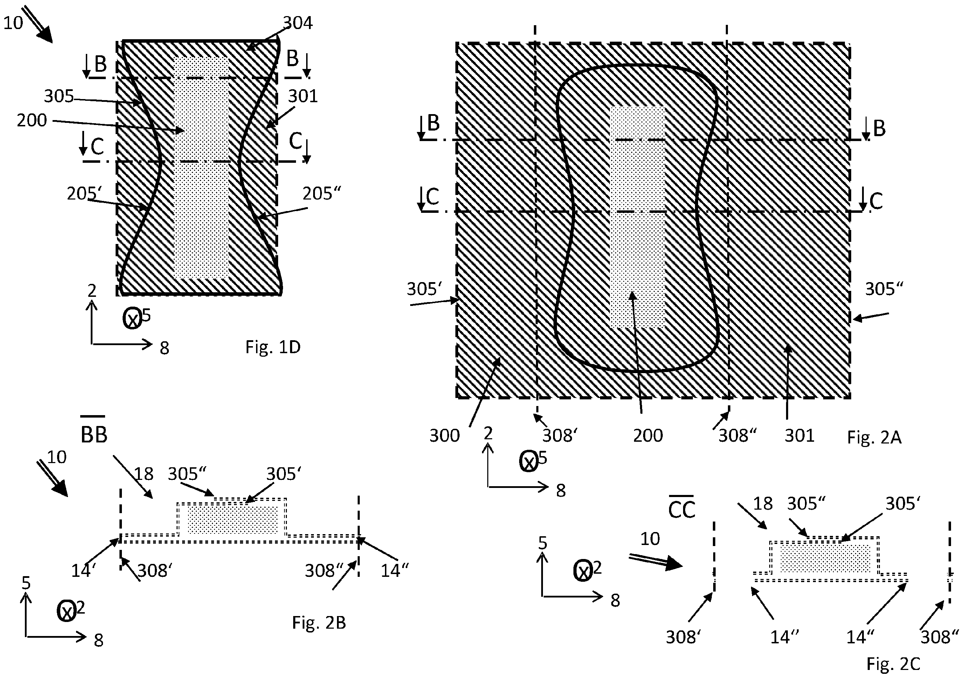

The envelope may comprise a user-oriented envelope web 304 and an opposite envelope web 306 which are shaped according to the shape of the absorbent pad 10, and are sandwiching the absorbent core 200 at least along its longitudinally extending side margins 205 and preferably also along the cross-directionally extending front (202) and rear (208) end margins. The envelope webs may be formed from envelope web precursor web. Fig. 1D shows a rectangular envelope web precursor 301 with dashed circumferential lines, from which the envelope web 306 is formed by removing the lateral side portions outside of the longitudinal side margins 205', 205" of the absorbent pad 10, at least in its crotch region 15. The webs may be made from the same or different material, for example, the user-oriented web 304 may have fluid handling enhancements, such as being more hydrophilic, or the opposite web 306 may have fluid repelling properties, such as by being more hydrophobic, or exhibiting smaller pores, e.g. by thinner filaments.

The webs are bonded to each other as may be achieved by conventional bonding means such as adhesive and/or thermofusion. However, as discussed hereinbelow, the envelope webs may be formed just prior to the combining and may thus exhibit sufficient thermal tackiness to bond to each other.

For this execution, the first and the second envelope do not necessarily be of the same type of material, such that the user-oriented web may be hydrophilized to enhance fluid penetration whilst the opposite web may be hydrophobic so as to minimize liquid wicking. Either one or each envelope web may be colored to enhance visual perception.

The envelope may also be formed from a single web which is overfolded along longitudinally extending side margins, such as in a "G-type" overfold, see Fig. 2A, showing an absorbent core 200 positioned on a wide precursor web 301. Upon overfolding along longitudinal fold lines 308', 308", the longitudinal margins of the envelope web 305', 305" overlap, see the cross-sectional vie in Fig. 2B, and may be connected to each other by conventional bonding means such as adhesive and/or thermofusion. Further the side portions may be removed and - in analogy to Fig. 1D - the shape of the absorbent pad with absorbent pad side margins 14 corresponds to the shape of the envelope webs with a narrower width in the crotch region 15. However, as discussed hereinbelow, the web may be formed just prior to the combining and may thus exhibit sufficient thermal tackiness to bond to itself in the overlap region.

It should be noted that the overlap region does not need to be along the longitudinal centerline of the core, but can be laterally offset, and can be on the user-oriented portion of the absorbent core or on the opposite side.

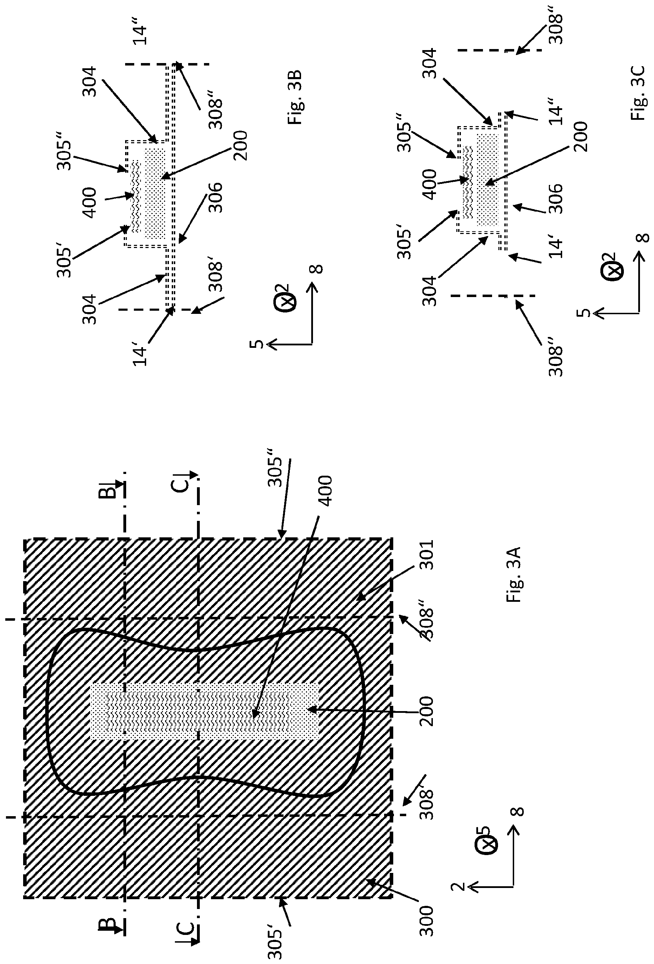

In referring to Fig. 3 A to C, a further alternative can be described as a "C-type" overfold, similar to the "G-type" except that the precursor envelope web 301 is not overfolded onto itself but the longitudinal margins just overlap an additional material 400 positioned on the absorbent core, which should exhibit containment function for the absorbent material but may also exhibit further properties such as improving fluid handling, which may the also be referred to as "acquisition distribution material". Also for this option, the tackiness of the envelop precursor may be sufficient, or additional bonding between the additional material and the envelope web and/or the absorbent core may be provided.

It should be noted, that also for the other options a suitable additional material may also be inserted between the absorbent core and the envelope - or even on top of the envelope.

In another aspect, the present invention is a process for forming an absorbent pad, which may be manufactured as an article as such or which may be further combined in downstream converter units with other elements, such as chassis elements like further webs as topsheet or backsheet, or elastic materials or closure means so as to form an absorbent article.

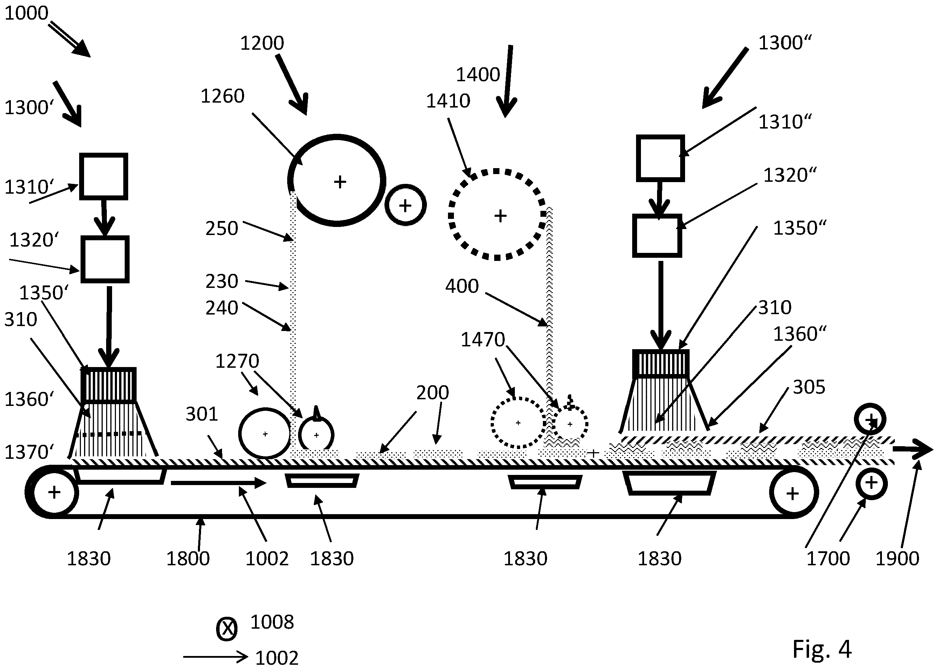

Such a process is further explained by referring to Fig. 4, showing an absorbent pad manufacturing unit 1000, comprising an absorbent core supply unit 1200 and at least a first continuous filament forming unit 1300' for forming a first envelope web precursor 301 from continuous polymeric filaments 310. A further continuous filament forming unit 1300" may provide a second envelope web precursor 302, which may be omitted if an overfolding unit 1600 closes the envelope. A shape forming unit 1700 forms the shape of the absorbent pad, e.g., but cutting such as die cutting.

The process is operated on at least one web support unit(s) 1800, defining the machine direction 1002 and the cross-machine direction 1008 of the process.

In a first step, an envelope web precursor 301 is formed by a first continuous filament forming unit 1300', comprising a first polymer supply 1310', a first polymer extruder 1320', and a first polymer extrusion die head 1350'. The die head 1350' is executed as a multi-row multicolumn filament expelling unit for forming essentially continuous micro-filaments 310, as described above.

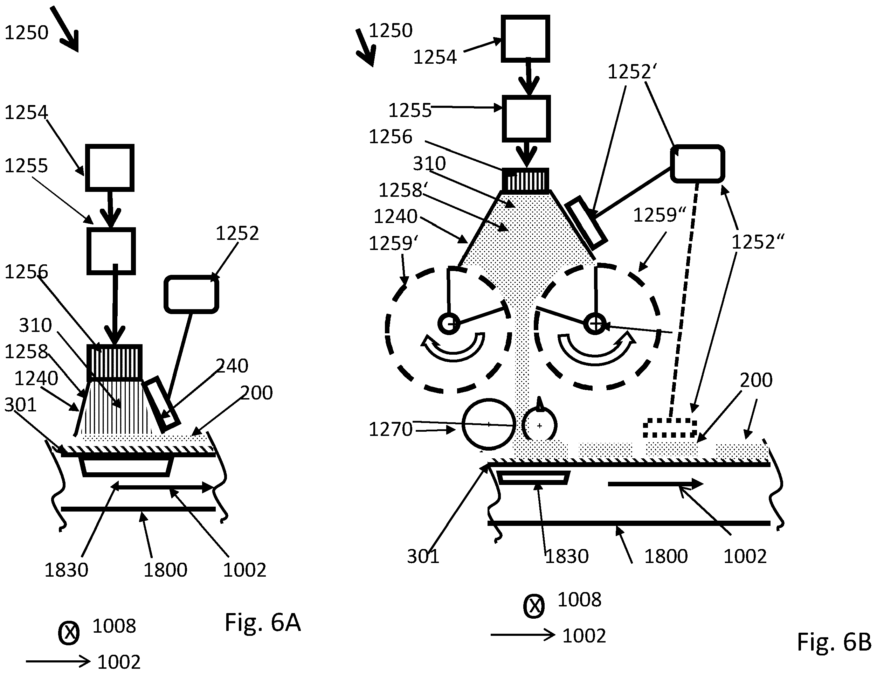

The essentially continuous filaments 310 are expelled and travelling - preferably within a forming box 1360 for separating the filament forming and cooling from the environment - towards the laydown region, which in Fig. 4 is part of the web transfer system 1800 and preferably supported by a vacuum system 1830. Instead of a flat laydown region, the filaments may be expelled towards a gap between two foraminous drums 1259, as described in the context of Fig. 6B herein below. Preferably, the filament forming unit is executed so as to allow start-stop-operation of the absorbent pad manufacturing unit 1000, especially if this unit is connected to further process units 1900 for forming absorbent articles, such as described in more detail in co-pending application EP24161946 (CoAx, unpublished), to which express reference is made as far as the intermittent operation and the diverting and recycling of filaments 310 in case of an abrupt interruption of the line is required. In a first execution, the die head 1350' provides a first envelop web precursor 301 at a constant width 307', and the shape of the absorbent pad is achieved by trimming away lateral portions further downstream.

In a second execution, the width 307' of the first envelope precursor 301 may vary ongoingly between a maximum width 307'(max) and a minimum width 307'(min), as described in the context of the absorbent pad in the above. Such a width adjustment may be achieved according to the teaching of EP24161914 (CoAx, unpublished), describing the adjustment of the width of a spray of continuous filaments by movable walls of a forming box 1360'.

The lateral side margins of the web may preferably be sharply defined according to the teaching of EP24161923 (CoAx, unpublished), describing the adjustable lateral baffle plates 1370, indicated in Fig 4 in dotted line mode.

After the first continuous filament envelope web precursor 301 is formed, it is transferred by the web transfer unit 1800, which may be unitary (as exemplarily shown) or comprise several sections and / or vacuum units 1830, towards the absorbent core supply unit 1200.

As exemplarily depicted in Fig. 4, the absorbent core supply unit 1200 may provide a preformed absorbent core web 250 as may comprise a mix of fibers 230 and SAP particles 240, as described in the above, and as may be supplied from a preformed web supply 1260, e.g., on a roll, spool, or a box. The preformed absorbent web may be sized in width and length and be cut into a series of individual absorbent cores 200, to be positioned spaced apart, e.g., by a cut and space unit 1270 onto the continuous filament envelope web 300 on the web transfer unit(s) 1800, optionally also supported by a vacuum unit 1830.

Optionally, and hence shown in dotted mode in Fig. 4, an additional material 400, e.g., a fluid handling layer, may be provided by an additional material supply unit 1400, as may comprise an additional material web supply unit 1410, cut into a series of individual additional web material pieces 400, to be positioned spaced apart, e.g., by a cut and space unit 1470 onto said absorbent core 200. Alternatively, the additional material web may be formed in-line from continuous micro-filaments in analogy to the web forming unit 1300.

In the next processing step, the absorbent core 200 - with the additional material 400, if present - is now enveloped by a continuous filament web 300.

In a first approach to enveloping, the composite is transferred to a second continuous filament envelop web forming unit 1300", where a second envelope web precursor 302 is applied in analogy to the first, optionally, though not necessarily, made from the same polymer as the first.

The shape of the absorbent pad 10 with a varying pad width along its longitudinal extension may then be formed by removing lateral portions of the envelope precursor 301, 302 by a cutting and separation unit 1700, such as conventional die cutter, to be transferred to the further processing unit(s) 1900. Preferably, the process further includes the recycling of the separated lateral portions, optionally recycling these into the filament forming unit (1300).

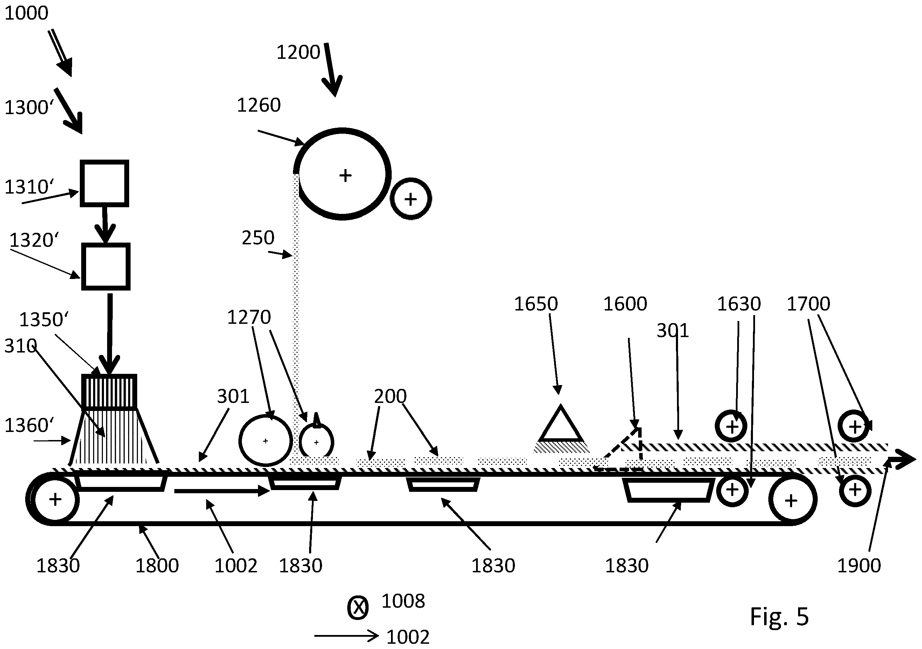

In an alternative approach, see Fig. 5, the precursor web 301 is formed at a width sufficient to cover all surfaces of the absorbent core 200 as depicted in Fig. 2A and/or of the composite of the absorbent core 200 and the additional material 400, if present. The enveloping may then be performed by a folding unit 1600, such as a conventional folding board, preferably followed by a compaction unit 1630.

As further indicated in Fig 5, for either of the approaches additional adhesive applicator 1650 may apply adhesive to further strengthen the bonding of the various elements, although this is not necessary if the precursor web still exhibits sufficient tackiness.

Optionally, the pads may also be cross-directionally separated to form a series of individualized pads. Optionally, additional compacting and/or bonding may be applied, such as by compaction rolls 1730, which may also apply ultrasonics.

Instead of employing a preformed absorbent core as described in the context of Fig. 4 and 5, it may be preferred to form an absorbent core in-line, see Fig. 6.

In a first approach for an in-line formed absorbent core, see Fig. 7A, the absorbent core supply unit 1250 comprises a continuous filament forming unit in analogy to the to the continuous filament web forming unit 1300 for the precursor web 301, although it may be operated with a different polymer and/or different process setting. To this end, an in-line core forming unit 1250 is provided, comprising
an SAP supply unit 1252;
a filament polymer supply unit 1254 with a core filament polymer;
a polymer extruder 1255;
a continuous filament forming unit 1256;
an SAP-Filament intermixing unit 1258.

The continuous polymeric filaments 310 from the core filament polymer are formed by a continuous filament forming unit 1256 and expelled into a SAP-Filament intermixing unit 1258, e.g., a forming box 1240, where these are intermixed with SAP particles 240 from the SAP supply unit.

The filament/SAP particle intermix is then deposited onto the first envelope web precursor 301, preferably supported by a suction unit 1830 of the web transfer system 1800.

A second approach for an in-line formed absorbent core, see Fig. 7B, may provide an advantage in that can provide more open webs, and also an easier approach for placing the absorbent cores machine directionally apart from each other onto the first precursor web 301.

Similarly, to the first approach, an in-line core forming unit 1250' is provided, comprising
an SAP supply unit 1252;
a filament polymer supply unit 1254 with a core filament polymer;
a polymer extruder 1255;
a continuous filament forming unit 1256;
an SAP-Filament intermixing unit 1258.

However instead of depositing the filament / SAP intermix directly onto a flat web transfer unit, it the filaments from the filament forming unit 1256 and the SAP from the SAP supply unit 1152' is now fed into a filament 7SAP intermixing unit 1258', e.g., within a forming box 1240 and into the gap between two foraminous drums 1259' and 1259", allowing to adjust the web density via the gap width and also to solidify the filaments to a larger extent, thus providing sufficient integrity to the intermix, such that it can be separated into individualized absorbent cores, such as by a cut and space unit 1270. More downstream, such an absorbent core may be processed as described in the above.

Yet a further alternative to the in-line forming of the absorbent core, as depicted in Fig. 6B in a dotted mode, is analogously to the second one, except that the SAP particles are not fed into the SAP filament intermixing unit 1258' but are applied by the SAP application unit 1252" after the absorbent core filament web is deposited onto the web support 1800 by sprinkling it onto the surface of this web, optionally in a phased pattern as may correspond to the individually spaced apart web pieces.

As a skilled person will readily realize, various options and alternatives, as have been described in a certain context may be combined with other alternatives described in a different context, e.g., an of the in-line core forming alternatives may be combined with any of the envelope forming options.

## Claims

1. An absorbent pad (10) for an absorbent article
comprising
an absorbent core (200)
exhibiting
- length (2), width (8) and thickness (5) extension;
- a user-oriented surface (204) and an opposite surface (206);
- a first and a second longitudinally extending core side margin (205', 205");
- a front (202) and a rear (208) cross-directionally extending core end margin;
and comprising superabsorbent polymer (SAP) particles (240) intermixed with fibers (230), preferably continuous polymeric filaments (231);
at least one core envelope web (300)
covering at least said user oriented (204) and said opposite surface (206) of said core (200), projecting laterally outwardly of said first and a second longitudinally extending core side margin (205', 205"), thereby
forming longitudinally extending core envelope web margins (305', 305")
and exhibiting a core envelope web width (308)
whereby said absorbent core envelope web is consisting essentially of continuous micro-filaments (310), preferably of the CoAx type,
**characterized in that**
said core envelope web width (308) is varying along the length extension (201) of said core (200).

2. An absorbent pad (10) for an absorbent article according to claim 1, further with one or more elements selected from the group consisting of
- said absorbent core (200) is enveloped by a single web overfolded around said longitudinal core side margins (305) and overlapping on said user oriented (204) or said opposite (206) surface;
- said absorbent core (200) is enveloped by a user oriented (304) and an opposite (306) surface web connected to each other at least laterally outwardly of said longitudinal core side margins (205) of said absorbent core (200);
- said absorbent core (200) exhibits an essentially rectangular shape;
- said absorbent core (200) exhibits an absorbent core length (201) that is shorter than the one of said envelope web(s) (300).

3. An absorbent pad (10) for an absorbent article according to claim 1 or 2, further comprising one or more elements selected from the group consisting of
- said envelope web(s) (300) comprise synthetic thermoplastic polymers, preferably selected from the group consisting of PP, PE, PES, or PVA, optionally colored;
- said absorbent core (200) comprises superabsorbent material, preferably particulate superabsorbent material;
- said absorbent core (200) comprises superabsorbent polymer (SAP) particles (240) selected from the group consisting of polyacrylate-, cellulose-, or starch-based materials;
- said absorbent core (200) is essentially free of cellulosic material;
- said absorbent core (200) comprises an intermix of essentially continuous thermoplastic filaments (231) and SAP particles (240).
- said absorbent pad (10) further comprises an additional material layer (400) positioned between said absorbent core (200) and said absorbent core envelope web (300), preferably positioned on said user-oriented surface (204) of said absorbent core (200).

4. A process (2000) for forming an absorbent pad (10) comprising an absorbent core (200) and at least one envelope web (300),
said process being operated along a machine direction (2002) and exhibiting a cross-machine direction (2008),
said process (2000) comprising the steps of
a - providing an absorbent pad forming unit (1000) comprising
at least one continuous filament web forming unit (1300');
at least one absorbent core supply unit (1200) for providing an absorbent core (200);
at least one web transfer unit (1800);
optionally an additional material layer supply unit (1410) for providing an additional material (400);
b - forming a first continuous filament web as a first precursor web (301) of an envelope web (300) and depositing it on a web transfer device (1800);
c - transferring said first precursor web (301) towards said absorbent core supply unit (1300);
d - providing an absorbent core (200) by said absorbent core supply unit (1200);
e - depositing said absorbent core (200) onto said precursor web (301) on a web transfer device (1800);
f - optionally providing an additional material layer (400) and depositing it onto said absorbent core (200);
g - forming an envelope (300) for said absorbent core (200) to form said absorbent pad (10) by
gla - applying a second continuous filament web as a second precursor web (302) and
positioning this onto said absorbent core,
or
g1b - overfolding said first continuous filament web as a first precursor web (301)
and
g2 - bonding said envelop webs to each other at least in portions laterally outward of said core (200);
h - feeding said absorbent pad (10) to further processing units (1900) for manufacturing an absorbent article,
**characterized in that** it further comprises a step of
z - adjusting the width (308) of said absorbent core envelope web (300) such that it varies along the length of said absorbent core (200) aligned with the machine direction (1002) of said absorbent pad forming unit (1000).

5. A process (2000) for forming an absorbent article (10) according to claim 4, wherein
said step z) of adjusting the width of said absorbent core envelope web (300) is executed
- by adjusting the width of said continuous filament web as a first and second precursor web (301, 302) during step b) and/or ga1)
and/or
- by separating lateral portions of said continuous filament web as a first and second precursor web (301, 302), preferably further including recycling of the separated lateral portions, more preferably recycling the separated lateral portions into the filament forming unit (1300).

6. A process (2000) for forming an absorbent article (10) according to claim 4 or 5, wherein said step d) of providing an absorbent core (200) comprises the steps of
d1a - providing a preformed core precursor (211) on a preformed core precursor supply unit (1210);
d1b - adjusting the size of said core precursor (211) to the predetermined size of said absorbent core.

7. A process (2000) for forming an absorbent article (10) according to any of claims 4 to 6, wherein said step d) of providing an absorbent core (200) comprises the steps of
d2a - providing a core forming unit (1250) comprising
- an SAP supply unit (1252);
- a filament polymer supply unit (1254);
- a polymer extruder (1255);
- a continuous filament forming unit (1256);
- an SAP-Filament intermixing unit (1258);
d2b - forming continuous polymeric filaments (310) from a filament polymer by said continuous filament forming unit (1256);
d2c - intermixing said continuous polymeric filaments with SAP particles (240) from said SAP supply unit (1252) in said SAP-filament intermixing unit (1258).
